# EUROPEAN PATENT SPECIFICATION

(11) **EP 2 274 262 B1**
(45) Date of publication and mention of the grant of the patent: **29.08.2018**
(21) Application number: 08720163.8
(22) Date of filing: 13.03.2008
(51) Int. Cl.: C07C 41/09, C07C 43/10

(54) **A PROCESS FOR THE PREPARATION OF PRIMARY ALKYL GLYCEROL ETHERS USEFUL AS BIOFUEL ADDITIVE FROM GLYCEROL**
VERFAHREN ZUR HERSTELLUNG VON ALS BIOBRENNSTOFFZUSATZ GEEIGNETEN PRIMÄREN ALKYLGLYCERINETHERN AUS GLYCERIN
PROCÉDÉ DE PRÉPARATION D ÉTHERS DE TYPE ALKYLGLYCÉROLS PRIMAIRES UTILES EN TANT QU'ADDITIF POUR BIOCARBURANTS DÉRIVÉ DU GLYCÉROL

(43) Date of publication of application: 19.01.2011
(73) Proprietor: Council of Scientific & Industrial Research, New Delhi 110 001 (IN)
(72) Inventor: Srinivas, Darbha, Pune 411 008 Maharashtra (IN); Ratnasamy, Paul, Pune 411 008 Maharashtra (IN); Saikia, Lakshmi, Pune 411 008 Maharashtra (IN)
(74) Representative: Bassil, Nicholas Charles
(86) International application number: PCT/IN2008/000144
(87) International publication number: WO 2009/113079

(56) References cited:
- US-A1- 2007 260 078

## Description

### Field of the invention

The present disclosure relates to a process of the preparation of biofuels or biofuel additives from glycerol. More particularly, it relates to a process for the preparation of glycerol ethers by etherification of glycerol with an alcohol using solid acid catalysts.

### Background of the invention

Glycerol is a by-product in the manufacture of biodiesel by transesterification of triglycerides with alcohols like methanol. In this process, a large amount of glycerol is produced that is not utilized as a part of the biofuel. At present, glycerol finds applications only in very small volume products in the pharmaceutical industry. Other grade uses of glycerol include mixing with animal manure to form a fertilizer, and mixing with feed for animals. The economics of production of biodiesel can be significantly improved if new uses of the large' amount of glycerol produced are found. It is desirable to discover processes that convert glycerol-containing streams from the transesterification of vegetable or animal oils into more value-added material to improve the economics of biodiesel production. Conversion of glycerol into glycerol ethers which can find applications as blending components in fuels like gasoline or diesel is one attractive option in view of the large demand for such blending components. Crude glycerol produced as a by-product in the production of biodiesel via transesterification of triglycerides is insoluble in biodiesel. For blending into biodiesel, glycerol has to be converted into a product boiling in the diesel range and, in addition, freely soluble in the biodiesel.

U.S. patents 6,174,501 and 6,015,440 claim a process for producing biodiesel fuel with reduced viscosity and cloud point below 32 Fahrenheit wherein triglycerides are reacted in a liquid phase reaction with methanol and a homogeneous basic catalyst, like NaOH₁ yielding methyl esters and a glycerol phase containing, mainly, glycerol and some residual methanol. The glycerol phase was passed through a strong cationic ion exchange to remove anions, resulting in a neutral product, which was flashed to remove methanol and then, reacted with an olefin, like isobutylene or isoamylene, in the presence of a strong acid catalyst to produce glycerol ethers. The glycerol ethers are then added back to the methyl esters of glycerol to provide an improved biodiesel. U.S. patent 5,476,971 describes reacting pure glycerol with isobutylene in the presence of an acid catalyst in a two-phase reaction to produce mono-, di- and tri-tertiary butyl ethers of glycerol. In the three patents mentioned, hereinabove, isobutylene is claimed as the etherifying agent. Since triglycerides are obtained from vegetable oils, like Soya bean oil, palm oil and rapeseed oil, and olefins like isobutylene and isoamylene are obtained from the catalytic cracking of petroleum fractions, it will be more desirable to use, as etherifying agents, chemicals that are available in the agro industry, like agro ethanol. US 2007/0260078 describes an integrated process for the manufacture of biodiesel from vegetable or animal oils using a solid, transesterification catalyst and a solid, etherification catalyst. Only biodiesel containing blending components that are not sourced from fossil fuels can truly be called "biofuels". Moreover, chemical compounds containing the tertiary alkyl carbon atoms, like isobutylene and the glycerol ethers made from olefins, like isobutylene or isoamylene, are less biodegradable than those containing only primary carbon atoms, like ethanol, butanol or the glyceryl ethers made from such alcohols. There is, hence, need for a procedure, which enables the use of a primary alcohol and preferably, a primary alcohol, which is also available from the agro industry, such as ethanol, to etherify the glycerol.

It is well known in the art of etherification that etherification reactions involving alcohols as the etherifying agents are more difficult to accomplish than those wherein olefins are used as the etherifying agents. In particular, etherfications involving olefins are less subject to equilibrium limitations than those using alcohols and wherein water is produced as a co-product. More particularly, the water formed during the transesterification can poison the acid sites, which are the active sites for the transesterification reaction. As a consequence, those catalysts, like cation exchange resins, which are quite effective in the etherification with olefins, are less effective when alcohols are used as etherifying agents. In addition, continuous removal of the water formed during the reaction is also beneficial in driving the reaction to the product side. Carrying out the etherification reaction at temperatures higher than the boiling point of water (100°C) is also beneficial since above 100°C, the water formed can be continuously removed from the catalyst. One drawback of cation exchange resins for their use in etherification reactions above 100°C is that these polymeric resins are structurally unstable above about 100 - 12O°C and, hence, undergo irreversible structural decomposition above these temperatures.

### Objectives of the invention

The main objective of the present disclosure is to provide a process for the preparation of glycerol derived ethers and used thereof as biofuels or additive in biofuels.

Another object is to provide a single-step process for the preparation of glycerol ethers from the glycerol fraction of the products in the transesterification of vegetable oils and animal fats.

Yet another object of the present disclosure is to produce glycerol ethers by etherification of glycerol fraction obtained in the transesterification of vegetable oil or fat with a C1 - C8 alcohol, over a solid catalyst, at moderate conditions and shorter reaction times.

### Summary of the invention

According to a first aspect, the invention provides a process for the preparation of primary alkyl glycerol ethers wherein the said process consists essentially of reacting ethanol with glycerol, wherein the molar ratio of ethanol to glycerol is 5:1, in the presence of a solid acid catalyst which is 5% wt of the total reaction volume, at a temperature of 100°C, for a period of 5 hrs in a closed autoclave and separating the desired glycerol ethers formed from the above said reaction mixture by a known method, wherein the solid acid catalyst used is solid phosphoric acid.

The glycerol used is optionally a glycerol obtained as byproduct in the transesterification of vegetable oil or fat with alcohol.

According to a second aspect, the invention provides a process for the preparation of primary alkyl glycerol ethers wherein the said process consists essentially of reacting ethanol with glycerol, wherein the molar ratio of ethanol to glycerol is 5:1, in the presence of a solid acid catalyst which is 5% wt of the total reaction volume, at a temperature of 100°C, for a period of 5 hrs in a closed autoclave and separating the desired glycerol ethers formed from the above said reaction mixture by a known method, wherein the solid acid catalyst used is silicoaluminophosphate which has been pretreated by activating at 350°C.

The glycerol used is optionally a glycerol obtained as byproduct in the transesterification of vegetable oil or fat with alcohol.

The present disclosure also provides a process for the preparation of primary alkyl glycerol ethers which comprises reacting a primary alcohol with glycerol, in a molar ratio of primary alcohol to glycerol in the range of 3:1 to 9:1, in the presence of a solid acid catalyst, at a temperature in the range of 60 to 300°C, for a period of 5-8 hrs in a continuous stirred tank reactor or at a weight hourly space velocity of about 0.2 h^{'1} in a fixed bed reactor and separating the desired glycerol ethers formed from the above said reaction mixture by known method.

The primary alcohol used in this disclosure may be selected from the group consisting of methanol, ethanol, butanol and octanol.

The molar ratio of primary alcohol to glycerol in this disclosure is preferably in the range of 4 : 1 to 6 : 1.

The solid acid catalyst used in this disclosure may be selected from the group consisting of alumina, aluminosilicate, silicoaluminophosphate, solid phosphoric acid, sulphated zirconium oxide, sulphonic acid or thiol-functionalised silica and cation exchange resin.

The aluminosilicate used in this disclosure may be selected from the group consisting of zeolite beta, zeolite Y and mordenite.

The cation exchange resin used in this disclosure may be Amberlyst-15.

The reaction temperature used in this disclosure may be preferably in the range of 60-150°C.

The reactor used in this disclosure may be selected from the group of consisting of continuous stirred tank reactor, reactive distillation reactor and continuous-flow fixed-bed reactor.

The glycerol used in this disclosure may be a glycerol obtained as byproduct in the transesterification of vegetable oil or fat with alcohol.

The glycerol-containing feedstock in this disclosure may contain in addition to glycerol, esters of glycerol and carboxylic acids.

The % conversion of glycerol in this disclosure may be in the range of 60-95% by weight.

The yield of glycerol obtained in this disclosure may be in the range of 50-100% by weight of glycerol used.

### Detailed description of the invention

The present disclosure describes a process for the manufacture of glycerol ethers and more particularly, to a process for the manufacture of glycerol ethers by etherifying glycerol with a primary alcohol using solid, acid catalysts. The process comprises contacting a mixture of a primary alcohol and a glycerol- containing feedstock with a solid catalyst in a reactor at a temperature in the range of 60 to 300°C and a pressure of 1 - 10 bars and separating out most of the formed glycerol ethers from the reaction mixture.

The glycerol- containing feedstock can be conveniently obtained from the products of the transesterification of the triglycerides provided from vegetable oils. The transesterification of glycerides with methanol to yield biodiesel is well-known in the prior art. Usually base catalysts like NaOH or KOH are used in the liquid, homogeneous phase are used. There are several drawbacks in the use of such catalysts: (1) If free fatty acids are present in the triglycerides (and they are present in most, if not, all the vegetable oils), then, a prior esterification has to be carried out to neutralize them since, otherwise, they will combine with and deactivate the base catalysts; (2) The sodium ions in the reaction product have to be removed in an environmentally unsatisfactory acid-neutralisation step (involving disposal of acid-sludges) or an expensive removal in a cation exchange column (and eventual disposal of the sodium salt). Solid catalysts, which can accomplish the transesterification reaction, will be highly beneficial. Our co-pending Indian patent applications: 2722/DEL/2005 and 1561/DEL/2005 describe such a process for the transesterification of vegetable oils with alcohols using solid catalysts.

The effluent from the transesterification of vegetable oils over solid catalysts described in more detail in our above mentioned co-pending Indian patent applications: 1561 /DEL/2005 and 2722/DEL/2005 contains two, non- miscible, separable liquid phases, a lighter non-polar layer containing mainly the alkyl (methyl or ethyl) esters of the fatty acids (the biodiesel fraction) and a polar, heavier liquid layer containing glycerol and un reacted alcohol (methanol or ethanol). The glycerol and alcohol-containing fraction, obtained after removal of the biodiesel fraction, by decantation, for example, can, after addition of alcohol, if needed, constitutes a suitable feedstock for the process of the present invention. It may be noted that the use of solid catalysts instead of the caustic alkali in the transesterification stage as taught in our above mentioned co- pending Indian patent applications: 2722/DEL/2005 and 1561 /DEL/2005 has eliminated the need for the caustic removal and acid wash stages in the preparation of the glycerol feedstock for the etherification process.

This glycerol and alcohol-containing feedstock, after adjusting the alcohol to glycerol molar ratio to be above 4, if needed, is next contacted with a solid acid catalyst in a reaction zone maintained at reaction conditions optimized for the etherification reaction. We have found that temperatures in the range of 60 to 300°C, depending on the nature of the triglycerides, are very suitable for this reaction. The reaction is carried out at the autogenous pressure of the alcohol at the reaction temperature. The reaction zone can be constituted by a continuous stirred tank reactor (CSTR) or a continuous fixed bed reactor. In the CSTR, a residence time of about 3 hours or more was adequate whereas in the continuous fixed bed reactor, a weight hourly space velocity, WHSV (defined as the weight in grams of total feed passed through the reactor per hour per gram of catalyst) of above 0.2 led to complete conversion of the glycerol to the various mono-, di- and triethers of glycerol. The relative proportions of the three ethers can be controlled by the molar ratios of the alcohol to glycerol. High ratios led to mainly the di- and triethers while monoethers predominated at lower ratios. The choice idf the alcohol was determined by the end - use of the glycerol ether. If the glycerol ether is to be used as a blending component in biodiesel, then, methanol and ethanol were the preferred alcohols while octanol was the alcohol of choice if the glycerol ethers were to be used as biolubricants. Primary alcohols were preferred over secondary and tertiary alcohols in view of the greater biodegradability of the former. Thus, if production of biodiesel is the target, methanol or ethanol was used both in the transesterification as well as the etherification reactions. On the other hand, if the production of biolubricants is the main aim, then, octanol was used for both the reactions. It may be noted that our abovementioned co-pending Indian patent applications: 2722/DEL/2005 and 1561 /DEL/2005 provide a process for the production of biodiesel using methanol/ethanol or biolubricants using normal octanol as the transesterifying alcohol.

The solid catalyst used in the process of the present disclosure can be any solid acid, which contains substantial amounts of Lewis or Bronsted acid sites on the surface. Examples of such solid acids include alumina, fluorided alumina, chlorided alumina, amorphous silicoalumina, solid phosphoric acid, zeolites like zeolite Y₁, beta, inordenite, medium pore zeolites, silicoalumino phosphates, divalent metal-containing alumino phosphates, sulfated zirconium oxide and acid-washed clays. The primary alcohol used in this disclosure is selected from methanol, ethanol, butanol, and octanol, preferably agro-ethanol obtained from the fermentation of agro products like sugarcane and containing significant amounts of water. The solid catalyst used in the present disclosure is a solid acid catalyst, preferably alumina, silicoalumina, an aluminosilicate molecular sieve, a silicoaluminophosphate molecular sieve, acid-washed clay, solid phosphoric acid or sulfated zirconium oxide.

The practice of the present invention is illustrated with the following examples.

### Reference Example 1

This example illustrates the preparation of glycerol ethyl ethers over gamma alumina catalyst. In a typical preparation, ethanol and glycerol, in a molar ratio of 5 : 1, were heated with gamma-alumina catalyst (5% wt of the total reaction mixture) in a closed autoclave at 100°C for 5 hours. The solid acid, catalyst - gamma alumina was procured from commercial sources. Before use in the etherification reaction, the solid catalyst was pre-treated and activated at 500°C by procedures recommended by the catalyst manufacturer and well- known to those skilled in the art. At the end of 5 hours, the solid catalyst was removed, and the glycerol ethers were separated from the unreacted glycerol, water and alcohol by a combination of extraction (with water) and distillation procedures. The products were analyzed by gas chromatography.

### Reference Example 2

This example illustrates the preparation of glycerol ethyl ethers over zeolite-beta catalyst (silica/alumina = 40; surface area = 550 m²/g, size = 0.1 - 0.5 micron). In a typical preparation, ethanol and glycerol, in a molar ratio of 5 : 1, were heated with zeolite-beta (5% wt of the total reaction mixture) in a closed autoclave at 100°C for 5 hours. The solid catalyst was pre-treated activating at 450°C. At the end of the reaction, the catalyst was removed, and the glycerol ethers were separated from the unreacted glycerol, water and alcohol by a combination of extraction (with water) and distillation procedures. The products were analyzed by gas chromatography.

### Reference Example 3

This example illustrates the preparation of glycerol ethyl ethers over zeolite-Y catalyst (silica/alumina = 8; surface area = 420 m²/g). In a typical preparation, ethanol and glycerol, in a molar ratio of 5: 1, were heated with zeolite-Y (5% wt of the total reaction mixture) in a closed autoclave at 100°C for 5 hours. The solid catalyst was pre-treated activating at 450°C. At the end of the reaction, the catalyst was removed, and the glycerol ethers were separated from the unreacted glycerol, water and alcohol by a combination of extraction (with water) and distillation procedures. The products were analyzed by gas chromatography.

### Example 4

This example illustrates the preparation of glycerol ethyl ethers over silicoalumino phosphate catalyst. In a typical preparation, ethanol and glycerol, in a molar ratio of 5:1, were heated with the catalyst (5% wt of the total reaction mixture) in a closed autoclave at 100°C for 5 hours. The solid catalyst was pre-treated activating at 350°C. At the end of the reaction, the catalyst was removed, and the glycerol ethers were separated from the unreacted glycerol, water and alcohol by a combination of extraction (with water) and distillation procedures. The products were analyzed by gas chromatography.

### Reference Example 5

This example illustrates the preparation of glycerol ethyl ethers over Amberlyst-15 cation exchange resin (strongly acidic, macroreticular resin with sulfonic acid functionality, Aldrich Co.). In a typical preparation, ethanol and glycerol, in a molar ratio of 5 : 1, were heated with amberiyst-15 (5% wt of the total reaction mixture) in a closed autoclave at 100°C for 5 hours. The resin was pre-treated with 3 M H₂SO₄ and then activating at 100°C prior to use. At the end of the reaction, the catalyst was removed, and the glycerol ethers were separated from the unreacted glycerol, water and alcohol by a combination of extraction (with water) and distillation procedures. The products were analyzed by gas chromatography.

### Example 6

This example illustrates the preparation of glycerol ethyl ethers over solid phosphoric acid. In a typical preparation, ethanol and glycerol, in a molar ratio of 5 : 1, were heated with solid phosphoric acid (5% wt of the total reaction mixture) in a closed autoclave at 100°C for 5 hours. At the end of the reaction, the catalyst was removed, and the glycerol ethers were separated from the unreacted glycerol, water and alcohol by a combination of extraction (with water) and distillation procedures. The products were analyzed by gas chromatography.

### Reference Example 7

This example illustrates the preparation of glycerol ethers from glycerol and agro-ethanol (water content 5%) over zeolite-beta catalyst (silica/alumina = 40; surface area = 550 m²/g, size = 0.1 - 0.5 micron). In a typical preparation, agro-ethanol (water content 5%) and glycerol, in a molar ratio of 5 : 1, were heated with zeolite-beta (5% wt of the total reaction mixture) in a closed autoclave at 100°C for 5 hours. The solid catalyst was pre-treated activating at 45O°C. At the end of the reaction, the catalyst was removed, and the glycerol ethers were separated from the unreacted glycerol, water and alcohol by a combination of extraction (with water) and distillation procedures. The products were analyzed by gas chromatography.

### Reference Example 8

This example illustrates the preparation of glycerol butyl ethers over zeolite-beta catalyst (silica/alumina = 25; surface area = 550 m²/g, size = 0.1 - 0.5 micron). In a typical preparation, butanol and glycerol, in a molar ratio of 5 : 1, were heated with zeolite-beta (7.5% wt of the total reaction mixture) in a closed autoclave at 6O°C for 8 hours. The solid catalyst was pre-treated activating at 450°C. At the end of the reaction, the catalyst was removed, and the glycerol ethers were separated from the unreacted glycerol, water and alcohol by a combination of extraction (with water) and distillation procedures. The liquid products were analyzed by gas chromatography.

### Reference Example 9

This example illustrates the preparation of glycerol butyl ethers over zeolite-beta catalyst (silica/alumina = 25; surface area = 550 m²/g, size = 0.1 - 0.5 micron). In a typical preparation, butanol and glycerol, in a molar ratio of 5 : 1, were heated with zeolite-beta (7.5% wt of the total reaction mixture) in a closed autoclave at 90°C for 8 hours. The solid catalyst was pre-trea activating at 450°C. At the end of the reaction, the catalyst was removed, and the glycerol ethers were separated from the unreacted glycerol, water and alcohol by a combination of extraction (with water) and distillation procedures. The liquid products were analyzed by gas chromatography.

The results of the etherification reaction with various catalysts described in Examples 4 and 6 and Reference Examples 1 to 3, 5 and 7 to 9 are listed in Table 1.

**Table 1**

| Example/ Reference Example | Glycerol Conversion, wt % | Yield of glycerol ethers, wt% of glycerol |
|---|---|---|
| 1 | 60 | 50 |
| 2 | 92 | 85 |
| 3 | 87 | 80 |
| 4 | 65 | 61 |
| 5 | 83 | 76 |
| 6 | 74 | 69 |
| 7 | 70 | 85 |
| 8 | 93 | 100 |
| 9 | 66 | 100 |

## Claims

1. A process for the preparation of primary alkyl glycerol ethers wherein the said process consists essentially of reacting ethanol with glycerol, wherein the molar ratio of ethanol to glycerol is 5:1, in the presence of a solid acid catalyst which is 5% wt of the total reaction volume, at a temperature of 100°C, for a period of 5 hrs in a closed autoclave and separating the desired glycerol ethers formed from the above said reaction mixture by a known method, wherein the solid acid catalyst used is solid phosphoric acid.

2. A process according to claim 1, wherein the glycerol used is optionally a glycerol obtained as byproduct in the transesterification of vegetable oil or fat with alcohol.

3. A process for the preparation of primary alkyl glycerol ethers wherein the said process consists essentially of reacting ethanol with glycerol, wherein the molar ratio of ethanol to glycerol is 5:1, in the presence of a solid acid catalyst which is 5% wt of the total reaction volume, at a temperature of 100°C, for a period of 5 hrs in a closed autoclave and separating the desired glycerol ethers formed from the above said reaction mixture by a known method, wherein the solid acid catalyst used is silicoaluminophosphate which has been pretreated by activating at 350°C.

4. A process according to claim 3, wherein the glycerol used is optionally a glycerol obtained as byproduct in the transesterification of vegetable oil or fat with alcohol.

## Patentansprüche

1. Verfahren zur Herstellung von primären Alkylglycerinethern, wobei das Verfahren im Wesentlichen aus dem Umsetzen von Ethanol mit Glycerin, wobei das Molverhältnis von Ethanol zu Glycerin 5:1 beträgt, in Gegenwart eines festen sauren Katalysators, der 5 Gew.-% des gesamten Reaktionsvolumens ausmacht, bei einer Temperatur von 100 °C über einen Zeitraum von 5 h in einem geschlossenen Autoklaven und dem Abtrennen der gewünschten Glycerinether aus der obigen Reaktionsmischung nach einer bekannten Methode besteht, wobei es sich bei dem verwendeten festen sauren Katalysator um feste Phosphorsäure handelt.

2. Verfahren nach Anspruch 1, bei dem es sich bei dem verwendeten Glycerin gegebenenfalls um ein bei der Umesterung von pflanzlichem Öl oder Fett mit Alkohol als Nebenprodukt erhaltenes Glycerin handelt.

3. Verfahren zur Herstellung von primären Alkylglycerinethern, wobei das Verfahren im Wesentlichen aus dem Umsetzen von Ethanol mit Glycerin, wobei das Molverhältnis von Ethanol zu Glycerin 5:1 beträgt, in Gegenwart eines festen sauren Katalysators, der 5 Gew.-% des gesamten Reaktionsvolumens ausmacht, bei einer Temperatur von 100 °C über einen Zeitraum von 5 h in einem geschlossenen Autoklaven und dem Abtrennen der gewünschten Glycerinether aus der obigen Reaktionsmischung nach einer bekannten Methode besteht, wobei es sich bei dem verwendeten festen sauren Katalysator um durch Aktivieren bei 350 °C vorbehandeltes Siliciumaluminiumphosphat handelt.

4. Verfahren nach Anspruch 3, bei dem es sich bei dem verwendeten Glycerin gegebenenfalls um ein bei der Umesterung von pflanzlichem Öl oder Fett mit Alkohol als Nebenprodukt erhaltenes Glycerin handelt.

## Revendications

1. Procédé de préparation d'éthers de glycérol d'alkyle primaire, ledit procédé étant essentiellement constitué de la réaction d'éthanol avec du glycérol, dans lequel le rapport molaire de l'éthanol au glycérol est 5:1, en présence d'un catalyseur acide solide qui est 5 % en poids du volume de réaction total, à une température de 100 °C, pendant une durée de 5 h dans un autoclave fermé et la séparation des éthers de glycérol souhaités formés à partir dudit mélange de réaction ci-dessus par un procédé connu, dans lequel le catalyseur acide solide utilisé est l'acide phosphorique solide.

2. Procédé selon la revendication 1, dans lequel le glycérol utilisé est facultativement un glycérol obtenu en tant que sous-produit dans la transestérification d'huile ou matière grasse végétale avec un alcool.

3. Procédé de préparation d'éthers de glycérol d'alkyle primaire, ledit procédé étant essentiellement constitué de la réaction d'éthanol avec du glycérol, dans lequel le rapport molaire de l'éthanol au glycérol est 5:1, en présence d'un catalyseur acide solide qui est 5 % en poids du volume de réaction total, à une température de 100 °C, pendant une durée de 5 h dans un autoclave fermé et la séparation des éthers de glycérol souhaités formés à partir dudit mélange de réaction ci-dessus par un procédé connu, dans lequel le catalyseur acide solide utilisé est un silico-aluminophosphate qui a été prétraité par activation à 350 °C.

4. Procédé selon la revendication 3, dans lequel le glycérol utilisé est facultativement un glycérol obtenu en tant que sous-produit dans la transestérification d'huile ou matière grasse végétale avec un alcool.
